# EUROPEAN PATENT APPLICATION

(11) **EP 2 151 505 A1**
(43) Date of publication of application: **10.02.2010**
(21) Application number: 08305448.6
(22) Date of filing: 05.08.2008
(51) Int. Cl.: C12Q 1/68

(54) **Method for determining a predisposition to basal cell carcinoma and for screening treatments thereof**

(71) Applicant: Institut Gustave Roussy, 94805 Villejuif Cédex (FR)
(72) Inventor: Valin, Alexandre, 94800, VILLEJUIF (FR); Magnaldo, Thierry, 75018, PARIS (FR)
(74) Representative: Gallois, Valérie

(57) **Abstract**

The present invention relates to methods for detecting a predisposition to a basal cell carcinoma and methods for screening a treatment of a basal cell carcinoma.

## Description

### Field of the Invention

The present invention relates to the dermatologic field and provide methods for determining a predisposition to basal cell carcinoma and for screening treatments thereof.

### Background of the Invention

Epidermal tumors include melanomas and non-melanoma skin cancers (NMSC). NMSC are the most prevailing cancers and represent 29% of all adult human cancers. NMSC comprise basal cell carcinoma (BCC) and squamous cell carcinoma (SCC). BCCs account for 80% of NMSC and are the commonest cancers in adult human. BCC and SCC both originate from keratinocytes but have different properties. One of the main characteristics of BCC is that they almost never form metastases, contrary to SCC. However BCC can be highly locally invasive.

Nevoid basal cell carcinoma syndrome (NBCCS) or Gorlin syndrome is a rare autosomal dominant pathology associated with predisposition to up to hundreds of BCCs. In NBCCS patients, as in the general population, ultraviolet exposure is a major risk factor for BCC development. However, these patients also develop BCCs in sun-protected areas of the skin, suggesting the existence of other mechanisms than ultraviolet mutagenesis for BCC predisposition in NBCCS patients. In this context, the investigators have studied the influence of dermal fibroblasts on the development of BCC from keratinocytes. Other clinical traits include developmental defects, such as falx cerebri calcification, palmoplantar pits, facial dysmorphism, bifid ribs, odontogenic keratocysts and in 3 to 5 percent of patients, predisposition to other cancers such as medulloblastoma. In 1996, mutations in the tumor suppressor gene *PATCHED* (*PTCH*) have been found to be associated to the NBCCS (Hahn et al, 1996, J. Biol. Chem. 271, 12125; Johnson et al, 1996, Science 272, 1668 ; WO97/43414).

The PATCHED protein is the receptor of the morphogen SONIC HEDGEHOG (SHH). When SHH binds to PATCHED, the latter releases its inhibitory effect on SMOOTHENED (SMO). SMO can then transduce the signal, leading to the nuclear migration of the glioma-associated oncogene transcription factors (GLI). Finally target genes are transcribed, including *GLI* transcription factors (*GLI1* and *GLI2*), *PTCH* itself, *CYCLIN D1, DPP...*

In the general population, the PATCHED/SHH pathway is constitutively and systematically activated in BCCs, as illustrated by the over-expression of the targets of the pathway: *PTCH* and *GLI1*. This activation occurs independently of the presence of SHH. Loss of control of the PATCHED/SHH pathway in the most prevailing human tumor underlines the importance of its role in skin homeostasis.

In order to develop models of the disease, transgenic mice have been generated. Several studies have consisted in over-expressing the activator/effectors of the pathway. The transgenic mice over-expressing SHH, GLI1 or GLI2 spontaneously develop tumors that resemble BCC. These observations lead to the hypothesis that any alteration of the PATCHED/SHH pathway mimicking a loss of function of the tumor suppressor gene *PTCH* could be responsible for the development of BCCs. However, to date, the mechanisms leading to the development of BCC in NBCCS patients and in the general population must be further documented.

Accordingly, there is a strong need to develop methods for detecting a predisposition to BCC and methods for screening compounds useful for treating or preventing BCC.

### Summary of the Invention

The present invention provides an expression signature for fibroblasts of NBCCS patients. It is thought that this expression signature is common with the carcinoma associated fibroblasts (CAF). This is why, based on this signature, the present invention provides methods for detecting a predisposition to BCC and methods for screening compounds useful for treating or preventing BCC.

Accordingly, the present invention concerns an *in vitro* method for detecting a predisposition to basal cell carcinoma in a human subject, said method comprising i) providing a biological sample of said human subject; ii) determining in said sample the amount of gene products for at least 11 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; iii) comparing the determined amounts to reference amounts, thereby detecting the human subject which has a predisposition to basal cell carcinoma.

In addition, the present invention concerns an *in vitro* method for screening or identifying a test compound useful for treating a basal cell carcinoma, comprising i) providing fibroblasts having an expression profile with the genes MMP1, MMP3, COL11A1, CXCL12, MGP, ANGPTL2, ANGPTL4, FGF7, GREM1, SFRP2, WISP2, ID2 and TNC over-expressed and the genes COL3A1, COL7A1, LAMA2, DKK3 and WNT5A under-expressed; ii) contacting a test compound with fibroblasts; iii) determining the expression level of the genes MMP1, MMP3, COL11A1, CXCL12, MGP, ANGPTL2, ANGPTL4, FGF7, GREM1, SFRP2, WISP2, ID2, TNC, COL3A1, COL7A1, LAMA2, DKK3 and WNT5A; and iv) selecting the test compound which decreases the expression of at least one gene selected in the group consisting of MMP1, MMP3, COL11A1, CXCL12, MGP, ANGPTL2, ANGPTL4, FGF7, GREM1, SFRP2, WISP2, ID2 and TNC and/or which increases the expression of at least one gene selected in the group consisting of COL3A1, COL7A1, LAMA2, DKK3, and WNT5A in the fibroblasts.

The present invention further concerns a kit comprising a set of detection means selected from the group consisting of a pair of primers, a probe and an antibody specific to at least 11 genes among the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; and a DNA chip comprising a solid support which carries nucleic acids that are specific to least 11 genes among the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.

### Brief Description of the Drawings

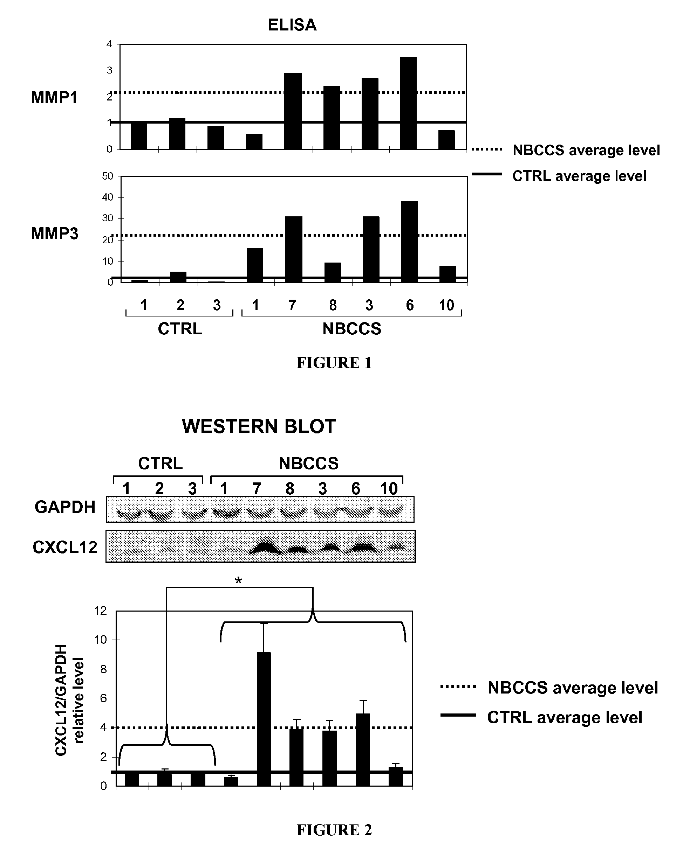

**Figure 1****. NBCCS fibroblasts in dermal equivalents over-express MMP1 and MMP3.** The levels of secreted MMP1 and MMP3 were determined by ELISA in the supernatants of the dermal equivalents used for the microarray assay. Results were set to 1 for the MMP levels in the supernatant of the dermal equivalent with control fibroblast strain 1. The average MMP levels in the supernatants of control (NBCCS) dermal equivalents are indicated with a plain (dotted) line.
**Figure 2****. Relative level of CXCL12 in WT and NBCCS fibroblasts.** Western blot analysis of CXCL12 was performed on cellular extracts of the 3 control and 6 NBCCS fibroblast strains. GAPDH was used as a loading control. The level of CXCL12 was normalized to the GAPDH level and was set to 1 in the control fibroblasts strain 1. A representative western blot and the average of 3 independent experiments are represented. Error bars refer to standard errors. The average CXCL12 level in control (NBCCS) fibroblasts is indicated with a plain (dotted) line (*: p≤0.05).

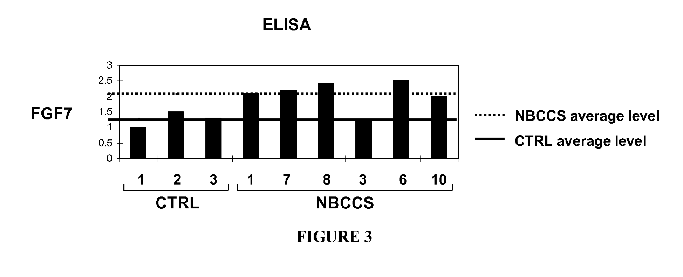

**Figure 3****. NBCCS fibroblasts in dermal equivalents over-express FGF7.** The levels of secreted FGF7 were determined by ELISA in the supernatants of the dermal equivalents used for the microarray assay. Results were set to 1 for the FGF7 level in the supernatant of the dermal equivalent with control fibroblast strain 1. The average FGF7 level in the supernatants of control (NBCCS) dermal equivalents is indicated with a plain (dotted) line.

### Detailed Description of the Invention

The present invention concerns an *in vitro* method for detecting a predisposition to basal cell carcinoma (BCC) in a human subject, said method comprising i) providing a biological sample of said human subject; ii) determining in said sample the amount of gene products for at least 11 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; iii) comparing the determined amounts to reference amounts, thereby detecting the human subject which has a predisposition to basal cell carcinoma.

Similarly, the present invention concerns an *in vitro* method for diagnosing whether a human subject is at risk of developing basal cell carcinoma (BCC), said method comprising i) providing a biological sample of said human subject; ii) determining in said sample the amount of gene products for at least 11 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; iii) comparing the determined amounts to reference amounts, thereby detecting whether the human subject is at risk of developing a basal cell carcinoma (BCC).

In the present document, the terms "a predisposition to" and "at risk of developing" are interchangeable and both can be used.

The present invention also provides a method for providing data for the detection of a predisposition to basal cell carcinoma (BCC) in a human subject, said method comprising i) providing a biological sample of said human subject; ii) determining in said sample the amount of gene products for at least 11 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; iii) comparing the determined amounts to reference amounts.

In a preferred embodiment of the methods of the invention, the method comprises determining in said sample the amount of gene products for at least 12, 13, 14, 15, 16, 17 or 18 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. In a particular embodiment, at least the amount of the following gene products is determined : MMP1, MMP3, COL11A1, LAMA2, CXCL12, MGP, ANGPTL2, ANGPTL4, FGF7, SFRP2 and DKK3. In a most preferred embodiment, the method comprises determining in said sample the amount of gene products for the following genes : MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.

Table 1, and in particular Tables 3, 4 and 5 indicate the list of genes having a modified expression when there is a predisposition to or a risk of developing basal cell carcinoma (BCC). In particular, the over-expression of the genes MMP1, MMP3, COL11A1, CXCL12, MGP, ANGPTL2, ANGPTL4, FGF7, GREM1, SFRP2, WISP2, ID2 and TNC and the under-expression of the genes COL3A1, COL7A1, LAMA2, DKK3, and WNT5A in comparison with a control fibroblast are indicative of a predisposition to or a risk of developing a basal cell carcinoma (BCC).

Accordingly, the methods of the invention can further comprise the expression level measurement of at least one, two, 5, 10, 15, 20, 25, 30, 35, 40, 45 or 50 additional genes selected in the group of the genes listed in Table 1, preferably in Table 3. In particular, the methods of the invention can comprise the expression level measurement of the genes of Table 1 or 3, preferably of Table 3.

More particularly, the methods of the invention can allow detecting a predisposition to sporadic BCC; and/or a familial predisposition to BCC, associated or not to NBCCS. In a particular embodiment, the methods of the invention are well-appropriate for detecting a familial predisposition to BCC unassociated with NBCCS. Alternatively, the present methods also allow detecting a predisposition BCC associated with NBCCS or a subject having a NBCCS.

This information is important because, if BCC has no impact on the life expectancy, the following of the patient allows an early operation with less esthetic impact for the patient.

In addition, the present invention also provides a method for discriminate between the different type of skin cancers. In particular, the detection of the NBCCS expression signature can allow detecting a predisposition to BCC and excluding a predisposition to SCC. This information can be important to the physician in order to select the appropriate following for the patient. Indeed, contrary to BCC, SCC can be associated to metastasis and therefore needs a narrow following of the patient.

The present invention also provides methods for screening, selecting or identifying a test compound useful for treating a basal cell carcinoma, comprising i) providing fibroblasts having an expression profile with the genes MMP1, MMP3, COL11A1, CXCL12, MGP, ANGPTL2, ANGPTL4, FGF7, GREM1, SFRP2, WISP2, ID2 and TNC over-expressed and the genes COL3A1, COL7A1, LAMA2, DKK3 and WNT5A under-expressed; ii) contacting a test compound with the fibroblast; iii) determining the expression level of the genes MMP1, MMP3, COL11A1, CXCL12, MGP, ANGPTL2, ANGPTL4, FGF7, GREM1, SFRP2, WISP2, ID2, TNC, COL3A1, COL7A1, LAMA2, DKK3 and WNT5A; and iv) selecting the test compound which decreases the expression of at one gene selected in the group consisting of MMP1, MMP3, COL11A1, CXCL12, MGP, ANGPTL2, ANGPTL4, FGF7, GREM1, SFRP2, WISP2, ID2 and TNC and/or which increases the expression of at one gene selected in the group consisting of COL3A1, COL7A1, LAMA2, DKK3, and WNT5A in the fibroblasts. By "treating" is intended a prophylactic or therapeutic treatment. The prophylactic treatment allows decreasing the number and the frequency of BCC in a subject or preventing the emergence of BCC. The therapeutic treatment allows curing the BCC or reducing the symptoms of BCC.

In a more preferred embodiment, the fibroblasts show a similar expression profile for the genes MMP1, MMP3, COL11A1, CXCL12, MGP, ANGPTL2, ANGPTL4, FGF7, GREM1, SFRP2, WISP2, ID2, TNC, COL3A1, COL7A1, LAMA2, DKK3 and WNT5A than the NBCCS expression signature. In addition to these genes, the expression profile for other genes of Table 1 or 3, preferably those of Table 3, can also be checked to be similar or identical to the NBCCS expression signature. By "NBCCS expression signature" is intended herein the pattern of over- or under-expression of the genes disclosed in Table 1, preferably in Table 3.

In a first embodiment, the fibroblasts provide from a skin biopsy, in particular a biopsy comprising fibroblasts, of a subject having the required NBCCS expression signature, in particular of a NBCCS subject. By "NBCCS subject" is intended a subject having one mutated allele of the *PATCHED* gene, in particular a missense or nonsense mutation. Such mutations have been disclosed in the patent application WO 97/43414. In a second embodiment, the fibroblasts are provided from genetic engineering of a fibroblast by introducing a mutation in the *PATCHED* gene.

The fibroblasts are then cultured in appropriate conditions well-known by the man skilled in the art. The fibroblasts can be cultured in a standard 2D culture. Alternatively, the fibroblasts can be cultured in a tridimensional type I collagen matrix called dermal equivalent. The fibroblasts can also be cultured in a three-dimensional organotypic skin system comprising a wild-type dermal equivalent. Organotypic skin system can be prepared essentially as detail in Asselineau et al, 1985 (Exp. Cell. Res., 159, 536-539) and Bernerd et al, 2001 (Proc. Natl. Acad. Sci. USA, 98, 7817-7822). The principle relies on the production of lattice in which fibroblasts are embedded in a type I collagen gel. After contraction of the lattice, keratinocytes are seeded thereon and allowed to overlay the dermal equivalent (the lattice). Optionally, the test compound is contacted with the fibroblasts through the keratinocytes layer.

Otherwise, the fibroblasts can be cultured in an *in vivo* skin regeneration, in particular by grafting the fibroblasts on an immunodeficient mouse. More precisely, organotypic skin system comprising the fibroblasts is grafted onto the nude mice either orthotopically or subcutaneously. For instance, the *in vivo* skin regeneration can be carried out as detailed in Del Rio et al, 2002 (Hum. Gene Ther., 13, 959-968).

The test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance. In particular, the test compounds can be a siRNA or antisense directed against over-expressed genes. The test compound is formulated in order to be in contact with the fibroblasts.

The method leads to the selection of test compound allowing to restore partially or completely the normal expression signature, in particular by decreasing the expression of the over-expressed genes and/or by increasing the under-expressed genes in the NBCCS expression signature. By "normal expression" is intended the expression of the gene observed in normal, control or wild-type fibroblasts. Preferably, the test compound restoring the normal expression of the genes of Table 5 is selected. The method can also comprise additional steps, in particular in an *in vivo* skin regeneration system, of determining the number and the frequency of BCC. It is thought that the restoration of a fully or partially corrected NBCCS expression signature in the fibroblasts decreases the risk of BCC development.

Determination of the expression level of a gene can be performed by a variety of techniques, from a biological sample. The term "biological sample" means any biological sample derived from a patient, preferably a sample which contains nucleic acids. Examples of such samples include fluids, tissues, cell samples, organs, biopsies, etc. Most preferred samples are samples comprising dermal fibroblasts, for instance a skin biopsy. The biological sample may be treated prior to its use, *e*.*g*. in order to culture the fibroblasts and/or to render nucleic acids or polypeptides available. Techniques of cell lysis, concentration or dilution of nucleic acids or proteins, are known by the skilled person.

Generally, the expression level as determined is a relative expression level. It is determined through the measure of the gene product amount in the sample and the comparison with a reference value. In a preferred embodiment, the reference value is the gene product amount determined in a healthy control sample. In this case, the over- or under-expression of the gene is assessed. In another embodiment, the reference value is the gene product amount determined in a sample of a subject having a predisposition or being at risk of developing BCC, in particular a subject having a familial predisposition to BCC, associated or not to NBCCS, more particular a NBCCS subject. In this case, the expression of the gene is similar or identical to the reference value. By "gene product" is intended the nucleic acid, (i.e., mRNA or microRNA) or polypeptide encoded by the gene. In addition, the amount of gene product for at least one control gene is determined in order to normalize the result. For instance, the control gene can be GAPDH.

More preferably, the determination comprises contacting the sample with selective reagents or detection means such as probes, primers or ligands, and thereby detecting the presence and measuring the amount of polypeptide or nucleic acids of interest originally in the sample. Preferably, the ligands are antibodies specific to the encoded polypeptide. Contacting may be performed in any suitable device, such as a plate, microtiter dish, test tube, well, glass, column, and so forth. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a particular embodiment, the expression level may be determined by determining the quantity of mRNA.

Methods for determining the quantity of mRNA are well known in the art. For example, the nucleic acid contained in the samples (*e*.*g*., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (*e*. *g*., Northern blot analysis) and/or amplification (*e*.*g*., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, *e*.*g*., 50 % formamide, 5x or 6x SCC. SCC is a 0.15 M NaCl, 0.015 M Na-citrate).

The nucleic acid primers or probes used herein may be assembled as a kit. Such a kit can include consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labeled and contacted with the microarray in hybridization conditions, leading to the formation of complexes between target nucleic acids that are complementary to probe sequences attached to the microarray surface. The labeled hybridized complexes are then detected and can be quantified or semi-quantified. Labeling may be achieved by various methods, *e*.*g*. by using radioactive or fluorescent labeling. Many variants of the microarray hybridization technology are available to the man skilled in the art.

Other methods for determining the expression level of said genes include the determination of the quantity of proteins encoded by said genes.

Such methods comprise contacting a biological sample with a binding partner capable of selectively interacting with a marker protein present in the sample. The binding partner is generally an antibody, that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. Also, the protein expression may be detected by immunohistochemistry on tissue section of the tumor sample (*e*.*g*. frozen or formalin-fixed paraffin embedded material). The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (*e*. *g.,* in membrane or microtiter well form); polyvinylchloride (*e*. *g.,* sheets or microtiter wells); polystyrene latex (*e*.*g*., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

The invention further provides a diagnostic tool for implementing said methods, e.g. a DNA chip comprising a solid support which carries nucleic acids that are specific to the cited genes from Tables 1, 3, 4 and 5, including at least 11, 12, 13, 14, 15, 16, 17 or 18 of the following genes: MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. Preferably, the diagnostic tool includes nucleic acids that are specific the following genes : MMP1, MMP3, COL11A1, LAMA2, CXCL12, MGP, ANGPTL2, ANGPTL4, FGF7, SFRP2 and DKK3; or MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. In addition, the present invention also provides a diagnostic kit for implementing said methods comprising detection means that are specific to the cited genes from Table 1, 3, 4 and 5, including at least 11, 12, 13, 14, 15, 16, 17 or 18 of the following genes: MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC, preferably including the following genes : MMP1, MMP3, COL11A1, LAMA2, CXCL12, MGP, ANGPTL2, ANGPTL4, FGF7, SFRP2 and DKK3; or MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. In particular, the detection means can be a pair of primers, a probe or an antibody. The kit can further comprise control reagents and other necessary reagents.

In this context, the invention further provides a DNA chip comprising a solid support which carries nucleic acids that are specific to at least 11, 12, 13, 14, 15, 16, 17 or 18 of the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC, preferably specific to at least the following genes : MMP1, MMP3, COL11A1, LAMA2, CXCL12, MGP, ANGPTL2, ANGPTL4, FGF7, SFRP2 and DKK3, more preferably specific to at least the following genes : MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. Chips which further carries nucleic acids that are specific to any or all of the genes listed in any of Table 1, or 4 are also useful in the present invention. The DNA chip can further comprise nucleic acids for control gene, for instance a positive and negative control or a nucleic acid for an ubiquitous gene in order to normalize the results.

The invention also relates to a kit comprising a set of detection means or reagents specific to at least 11, 12, 13, 14, 15, 16, 17 or 18 of the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC, preferably specific to at least the following genes : MMP1, MMP3, COL11A1, LAMA2, CXCL12, MGP, ANGPTL2, ANGPTL4, FGF7, SFRP2 and DKK3, more preferably specific to at least the following genes : MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC. Preferably, the detection means or reagents are selected from the group consisting of a pair of primers, a probe and a ligand, preferably an antibody.

The present invention also relates to the use of a DNA chip or a kit of the invention for preparing a diagnostic or screening kit for detecting a predisposition to or a risk of developing a BCC in a human subject.

The example illustrates the invention without limiting its scope.

### Examples

The inventors hypothesized that NBCCS fibroblasts could be involved in BCC predisposition. WT (wildtype) and NBCCS fibroblasts were cultured in a dermal equivalent model and their transcriptome were compared using a whole genome microarray analysis.

### RESULTS

### Whole genome microarray comparison of control and NBCCS fibroblasts transcriptomes

The inventors compared the genome wide expression profile of 9 primary independent fibroblasts strains cultured in dermal equivalents (CTRL, n=3; NBCCS, n=6). Three NBCCS fibroblast strains harbor missense mutations and 3 harbor nonsense mutations in the *PTCH* gene. They extracted total RNA from the dermal equivalents and gathered them in 3 pools according to the genetic status of *PTCH* (WT; missense and nonsense *PTCH* mutations). The transcription profile of the control pool was compared to the one of each NBCCS pool, on Agilent® human whole genome oligo microarray. Results were analyzed with the Rosetta Resolver® system for gene expression analysis.

To select the genes with a high probability to be differentially expressed in the NBCCS pools, the inventors set 10^-5 as the threshold for the p-value. With this criterion, 288 genes were found up or down-regulated (p-value<10^-5) in both the missense and the nonsense pools (included in Table 1). Among them, some genes were up-regulated and other genes were down-regulated in NBCCS fibroblasts (Table 1). The genes of the common signature displayed a high correlation between the two pools (correlation coefficient of 0.929). Only 6 probes corresponding to 5 genes were anti-correlated, i.e. up-regulated in one NBCCS pool and down-regulated in the other (CILP, LY6K, CLEC3B, CYP1B1 and LEPROT; Table 2). For all the primary sequences, the logarithm of the ratio of the intensities in each NBCCS pool and the WT pool was determined (log(ratio)). An analysis of variance (ANOVA) of the log(ratio) for all the primary sequences of the microarray was performed. Cluster analysis was done on the ANOVA results. For the first time in the field, observations demonstrate that missense or nonsense mutations of *PTCH* have overall very similar consequences on the transcriptome of dermal fibroblasts, in good agreement with the absence of genotype-phenotype relation in NBCCS patients.

The inventors have more precisely focused on the genes whose mRNAs amounts were increased at least twofold in both the missense and the nonsense pools or significantly decreased (included in Table 3).

### Over-expression of pro-tumoral matrix metalloproteinases and modified expression of extracellular matrix components in NBCCS fibroblasts

By applying SBIME (Searching for a Biological Interpretation of Microarray Experiments) (Kauffmann et al., 2008, Oncogene, 27, 565) to the data with the Gene Ontology annotations, the inventors found that the genes involved in the extracellular matrix composition or remodeling and in the cell adhesion were overrepresented among the genes with mRNA differentially expressed between control and NBCCS pools. Among all these mRNAs, the matrix metalloproteinase 3 (MMP3; stromelysin 1) mRNA exhibited the highest increased level in NBCCS pools. MMP3 mRNA level was found to be increased by 19.7 and 13 in the missense and the nonsense pools, respectively, compared to the control pool (Table 3). Similarly the matrix metalloproteinase 1 (MMP1; collagenase 1) mRNA level was increased by 8.4 and 7.8 (Table 3).

To confirm the microarray results, reverse transcription and quantitative polymerase chain reactions (RT-QPCR) were performed on each mRNA samples extracted from the 3 control and 6 NBCCS fibroblast primary strains used in the genomic screen. The average MMP1 and MMP3 mRNA levels were found statistically increased by 8.5 and 27, respectively, in NBCCS compared to control fibroblasts (p<0.025 for both of them) (Table 4). The inventors also measured by ELISA the level of MMP1 and MMP3 secreted proteins in the supernatants of the control and NBCCS dermal equivalents. The average levels of MMP1 and MMP3 were increased by 2.1 and 10.9 respectively, in the supernatants of NBCCS dermal equivalents compared to the average level in the supernatant of control dermal equivalents (Figure 1).

The mRNAs of some of the components of the extracellular matrix (ECM) were also found up-regulated in the missense and the nonsense pools. For instance the amount of collagen type 11 alpha 1 (COL11A1) mRNA was increased by 9.8 and 5.5 and Tenascin C (TNC) mRNA was increased by 1.4 and 1.3 in the missense and the nonsense pools respectively (Tables 1 and 3). RT-QPCR confirmed the increased level of COL11A1 mRNA in 5 of the 6 NBCCS fibroblasts compared to control fibroblasts. The average rate of COL11A1 mRNA over-expression in NBCCS fibroblasts is 11.5 (Table 4). Immunohistochemistry performed on organotypic skin cultures with control and NBCCS fibroblasts confirmed the over-expression of TNC by NBCCS fibroblasts.

Together these results indicate that some ECM components are expressed differentially between NBCCS and control fibroblasts.

### Under-expression of basement membrane components in NBCCS fibroblasts

The mRNA levels of some components of the basement membrane were decreased in the missense and the nonsense pools. Collagen type 3 alpha 1 (COL3A1) were decreased by 1.4 and 1.5 in the missense and the nonsense pools, respectively, compared to the control pool (Table 1). Similarly the collagen type 7 alpha 1 (COL7A1) mRNA level was decreased by 1.8 and 1.3 (Table 1). The mRNA level of the laminin alpha 2 was also decreased by 1.5 and 1.4 respectively in the missense and the nonsense pool (Table 1).

RT-QPCR confirmed the decreased average mRNA amounts of COL3A1, COL7A1, and LAMA2, by 1.4, 1.7 and 2.5 respectively (p≤0.05; p≤0.05; p<0.025), in NBCCS compared to control fibroblasts.

### NBCCS fibroblasts over-express the BMP antagonist GREMLIN1, growth factor and cytokines associated with BCC stroma

The mRNA levels of chemokine (C-X-C motif) ligand 12 (CXCL12 ; stromal-cell derived factor 1 alpha) and of the bone morphogenetic protein (BMP) antagonist GREMLIN1 (GREM1) were found up-regulated in inventors' microarray analysis, as well as those of Angiopoietin like 2 and 4 (ANGPTL2; ANGPTL4), matrix GLA protein (MGP) and fibroblast growth factor 7 (FGF7; keratinocyte growth factor). The respective mRNA level increases, in the missense and the nonsense pools, were 2.8 and 2.6 for CXCL12, 7.5 and 3.1 for ANGPTL4 (Table 3), 2 and 1.4 for MGP, 2 and 1.7 for ANGPTL2, 1.9 and 1.7 for GREM1 and 2.3 and 1.6 for FGF7 (Table 1).

RT-QPCR confirmed that CXCL12, MGP, ANGPTL2, ANGPTL4 and FGF7 average mRNA levels were increased in NBCCS fibroblasts by 2.4, 5.2, 3, 9.7, and 2.2 respectively (p<0.025 for each; Table 4). ANGPTL4 mRNA level was higher in fibroblasts of the missense pool than in fibroblasts of the nonsense pool. The increase of GREM1 mRNA level in NBCCS fibroblasts did not reach statistical significance.

These mRNA over-expressions were correlated with increased level of proteins as confirmed by western blot analysis on cellular extracts for CXCL12 (4.4 average fold increase; p≤0.05) (Figure 2) and by ELISA on the supernatant of dermal equivalents for FGF7 (1.7 fold increase) (Figure 3).

### The WNT/beta catenin pathway in NBCCS fibroblasts

The WNT/beta catenin pathway plays a crucial role in the regulation of proliferation and differentiation of keratinocytes (Slavik et al., 2007, *BMC Dev Biol*, 7, 9). In BCCs, nuclear beta catenin staining, corresponding to pathway activation, has been observed and associated with increased proliferation (Saldanha et al., 2004, Br J Dermatol, 151, 157, Yamazaki et al., 2001, Br J Dermatol, 145, 771). Several secreted inhibitors of the WNT/beta catenin pathway were either up or down-regulated in one or both of the NBCCS pools. Among them Dickkopf 1 (DKK3), secreted frizzed related protein 1 (SFRP1), SFRP2, WNT inhibitory factor 1 (WIF1) were up-regulated and DKK3 was down-regulated (Table 1). RT-QPCR indicated that the increased level of SFRP2 mRNA (3.5 fold increase; p<0.025) and the decreased level of DKK3 mRNA (3.9 fold decrease; p<0.05) in NBCCS fibroblasts were statistically significant (Table 4). Additionally, WNT5A, a ligand of the non canonical WNT pathway, was down-regulated by 2.9 and 2.7 in the missense and the nonsense pool respectively (Table 1). A significant decreased level in NBCCS fibroblasts was confirmed by RT-QPCR (average 2.0 fold decrease; p≤0.05; Table 4).

The mRNA levels of two target genes, WNT1 inducible signaling pathway protein 2 (WISP2) and inhibitor of DNA binding 2 (ID2) were increased in the NBCCS pools. WISP2 mRNA amount were increased by 2.5 and 2.1 in the missense and the nonsense pools respectively (Table 3) and ID2 mRNA amounts were increased by 2.3 and 1.6 (Table 1). We confirmed by RT-QPCR that WISP2 and ID2 mRNA levels were statistically increased (p≤0.05 and p<0.025 respectively) in NBCCS fibroblasts with the respective average fold increases 2.7 and 2.6 (Table 4).

### DISCUSSION

The study of carcinoma development has long been mainly focused on tumor suppressor genes mutations and proto-oncogenes activations in tumor cells. However, increasing evidences have indicated that tumor/stroma interactions regulate tumoral growth and invasiveness, and contribute to metastasis. In the case of BCCs, the stroma seems to be essential for the survival of cancer cells. By contrast to squamous cell carcinoma (SCC) cells, BCC cells can hardly be cultured *ex vivo,* and exhibit virtually null metastatic potential *in vivo.* Grafting experiments have shown that growth of BCC cells depends on the presence of their carcinoma associated fibroblasts (CAF). In this study, using whole genome transcriptome analyses, the inventors have investigated the extent to which *PTCH1*^{+/-} dermal fibroblasts could contribute to BCC development providing that NBCCS tumors also develop in non photo-exposed skin areas, i.e. in the absence of external genotoxic stress. Although isolated from healthy skin of patients, the inventors report here that NBCCS fibroblasts exhibit features very close to those of BCC CAF from non NBCCS individuals.

Perhaps, the most intriguing observation of this genomic screen was that none of the members of the SHH pathway was differentially expressed in NBCCS compared to control fibroblasts. In vertebrates, skin homeostasis relies on a balanced dialog between dermal and epidermal cells. Experimental models suggest that the SHH pathway not only is involved in the development and cycle of hair follicles but also in the regulation of recruitment and proliferation of interfollicular epidermal stem cells. Loss of control and activation of the SHH pathway was found in all BCCs tested. Thus, beyond its evident role in the control of growth of ectodermal cells, proper control of the SHH pathway could also contribute to dermo-epidermal interactions an hypothesis fitting well with distal transmission of the SHH signal between expressing and receiving cells and with growth features of BCC cells, as well.

As for members of the SHH pathway, quantification of mRNA amounts of members of the WNT pathway did not contribute to a clear conclusion on either activation or inhibition of the WNT pathway in NBCCS fibroblasts. Increased mRNAs levels of *SFRP2* and *WNT5A,* have been found in CAF of BCC (Micke et al., 2007, J Invest Dermatol, 127, 1516) and in whole BCC (O'Driscoll et al., 2006, Mol Cancer, 5, 74) respectively, and they were also found increased in the inventors' screen. In contrast, amount of *DKK3* mRNA which encodes another inhibitor of the WNT pathway was decreased (Table 4). Some target genes of the WNT pathway, *WISP2* and *ID2* were up-regulated (Table 4) while others such as *C-MYC* and *CYCLINDI* were not. *WISP2* has been shown to be strongly expressed in the stroma of breast tumors in Wnt1-transgenic mice (Pennica et al., 1998, Proc Natl Acad Sci U S A, 95, 14717). ID2 was over-expressed in human colorectal carcinomas (Rockman et al., 2001, J Biol Chem, 276,45113).

The absence of epidermal cells in the experimental settings may perhaps explain why, first, members and target genes of the SHH pathway were not found differentially expressed in NBCCS compared to control fibroblasts and second, the non-conclusive pictures drawn from study of the WNT pathway. Rather, the inventors' screening strategy may reflect cell autonomous impact of heterozygous *PTCH1* mutations in mesenchymal cells. Future experiments in the presence of both fibroblasts and keratinocytes should shed light on these issues.

For the first time, comparison of the whole genome expression in NBCCS and control fibroblasts clearly reveals a signature including several genes known for their association with tumor growth and invasiveness. A previous study reported over-expression of *MMP3* but not of *MMP1* mRNAs in cultured NBCCS fibroblasts and NBCCS BCCs (Majmudar et al., 1994, Mol Carcinog, 11, 29). Here, increases in MMP3 and MMP1 mRNAs and proteins amounts, were reported in NBCCS fibroblasts cultured in dermal equivalents (Table 4 and Figure 1). Interestingly over-expression of these MMPs has been reported in carcinoma associated fibroblasts (CAF) of sporadic BCCs (Monhian et al., 2005, Arch Facial Plast Surg, 7, 238, Majmudar et al., 1994, Mol Carcinog, 9, 17). Since, first, MMP1 processing is in part dependent on MMP3, second, these MMPs present partially distinct substrate specificities, and, third, none of the tissue inhibitors of metalloproteinase was found over-expressed, concomitant increases of MMP1 and MMP3 in patients fibroblasts strongly argue for a prominent role of the mesenchyme in BCC development and invasiveness in NBCCS. Epidermal over-expression of MMP1 in transgenic mice induces epidermal hyperplasia and increases chemical carcinogenesis (D'Armiento et al., 1995, Mol Cell Biol, 15, 5732). Also, MMP3 over-expression in mouse mammary epithelial cell line stimulates the epithelial to mesenchyme transition and invasiveness (Lochter et al., 1997, J Cell Biol, 139, 1861). Most interestingly, recent report from Dr. Bissell's laboratory indicated that MMP3-induced epithelial to mesenchyme transition and genomic instability are mediated through increased production of reactive oxygen species (Radisky et al., 2005, Nature, 436, 123). Thus, in NBCCS patients, MMP3 over-expression could well contribute to BCC development.

NBCCS fibroblasts also over-expressed the pro-tumoral ECM components TNC. TNC was found strongly expressed in the stroma of BCCs (Stamp, 1989, J Pathol, 159, 225) and TNC can up-regulate *MMP3* expression (Nishiura et al., 2005, Gynecol Endocrinol, 21, 111). Interestingly, TNC is a proteolysis substrate of MMP1 and MMP3 (Imai et al., 1994, FEBS Lett, 352, 216). In addition, TNC contains EGF-like repeats that binding to the EGF receptor stimulate mitogenesis (Swindle et al., 2001, J Cell Biol, 154, 459). The present study also shows that mRNA amounts of *CXCL12, GREM1* and *FGF7* (Table 4, Figure 2 and Figure 3) were also significantly increased in NBCCS fibroblasts. CXCL12 is involved in the invasion of BCC cells (Chu et al., 2007, Oncogene, 26, 2491) and can also recruit endothelial cells (Orimo et al., 2005, Cell, 121, 335, Salcedo et al., 2003, Microcirculation, 10, 359). GREM1 over-expression is associated with BCC stromal cells and promotes BCC cells proliferation (Sneddon et al., 2006, Proc Natl Acad Sci U S A, 103, 14842). FGF7 (keratinocyte growth factor) has a mitogenic activity in keratinocytes (Rubin et al., 1989, Proc Natl Acad Sci U S A, 86, 802). In NBCCS patients, presence of high levels of MMP1 and MMP3, as well as growth regulatory factors such as TNC, CXCL12, GREM1 and FGF7 could stimulate proliferation of BCC cells and elicit invasiveness. COL11A1 mRNA was also found increased in NBCCS fibroblasts, an observation reminiscent of its overexpression by stromal fibroblasts in human colorectal tumors (Fischer et al., 2001, Carcinogenesis, 22, 875). In contrast lower amounts of mRNAs of the basement membrane components *COL3A1, COL7A1* and *LAMA2* (Table 2) could be responsible for loosening basement membrane and, hence, facilitate local aggressiveness of pretumoral (*PTCH1*^{+/-} keratinocytes) or tumoral epidermal cells (Brellier Oncogene 2008).

Finally, increased *ANGPTL2* mRNA (3 times), which encodes a member of the angiopoietin-like family, could reflect proangiogenic properties as suggested by its effects on endothelial cells sprouting (Kim et al., 1999, J Biol Chem, 274, 26523). However, it was noteworthy that one of the most over-expressed (9.7 times) mRNA by NBCCS fibroblasts in this study was *ANGPTL4* (Table 3 and Table 4). ANGPTL4 can inhibit tumor cells (B16F0) motility and invasiveness (Galaup et al., 2006, Proc Natl Acad Sci U S A, 103, 18721) and exhibit anti-angiogenic properties (Ito et al., 2003, Cancer Res, 63, 6651). Whether inferior vascularization (Chin et al., 2003, J Pathol, 200, 308) and very low metastatic potential of BCC compared to SCCs could be related to high levels of ANGPTL4 requires further investigations. The inventors' data are in excellent agreement with those recently obtained by transcriptome analyses of BCC CAFs compared to perifollicular dermal fibroblasts (Micke et al., 2007, J Invest Dermatol, 127, 1516). In this study, the authors found that *SFRP2, ANGPTL2,* and *CXCL12* mRNA amounts were increased in CAF of BCC.

In summary, even if the causal link between *PTCH1*^{+/-} genetic status and abnormal expression of genes reported here remains to be clarified, these data clearly converge towards a global trend of NBCCS dermal fibroblasts to express some characteristics of CAF from BCCs. Modified expressions of ECM and basement membrane components, which are also observed in the stroma of BCCs (Howell et al., 2005, *J Dermatol Sci,* 39, 39), suggest that dermal fibroblasts might also produce a BCC facilitating microenvironment in NBCCS patients. Importantly, all primary fibroblasts used in this study were isolated from biopsies of healthy and photo-protected skin and gave very similar results irrespective of the type of *PTCH1* mutation. Altogether, the inventor's results strongly suggest the involvement of dermal fibroblasts in BCC predisposition in NBCCS patients including in non photo-exposed skin areas. On the other hand NBCCS keratinocytes form the same NBCCS patients exhibit spontaneous invasive properties in dermal equivalents populated with control fibroblasts (Brellier et al Oncogene 2008). More investigations must now be carried out in the long term to determine the extent to which NBCCS fibroblasts may affect fate of epidermal keratinocytes. Future studies should lead to improved approaches, taking into account the role of fibroblasts, in BCC prevention and/or treatment, not only in NBCCS patients but also in the general population.

### MATERIAL & METHODS

### Patients and cells

Skin biopsies from NBCCS and control persons were taken from sun-protected healthy areas of the skin. Control persons 1, 2 and 3 have no mutation in *PTCH1* alleles; NBCCS patients 1, 7 and 8 harbour independent missense mutations in *PTCH1*; NBCCS patients 3, 6 and 10 harbour nonsense independent mutations in *PTCH1*. NBCCS patients were described in (Brellier et al, 2008, Br J Dermatol). From these patients, primary dermal fibroblasts were cultured as described before (Rheinwald, 1975, Cell 6, 331). Experiments were performed using cells at passages 5 to 9.

### Dermal equivalents

In an effort to be as close as possible to the environment of fibroblasts in the dermis, all fibroblasts (WT n=3; NBCCS n=6) were cultured in a tridimensional type I collagen matrix called dermal equivalent. Dermal equivalents were cultured in immersion and then at the air-liquid interface as described in the organotypic skin model, except that keratinocytes were replaced by culture medium (Asselineau et al., 1985, Exp Cell Res, 159, 536).

### RNA extraction

Dermal equivalents were snap frozen in liquid nitrogen, ground to powder and then solubilized in TRIzol reagent (Invitrogen, Carlsbad, USA, CA). Then chloroform was added and the aqueous phase was removed. Total RNA were precipitated in isopropanol and wash twice before being resuspended in nuclease free water. Total RNAs were purified using the RNA cleanup and concentration kit (QIAGEN, Hilden, Germany) and gathered in 3 pools according to the genetic status of *PTCH1* (WT; missense and nonsense *PTCH* mutations).

### Whole genome microarray analysis

The mRNA pools were labeled using fluorescent low input linear amplification kit according to the manufacturer's protocol (Agilent, Santa Clara, USA, CA). Briefly, reverse transcription was performed using MMLV reverse transcriptase. Then, cyanine 3 or 5 labeled cRNAs were generated using T7 RNA polymerase. Hybridizations were carried out for 17 hours at 60°C with 1µg of purified control and NBCCS probes on Agilent® human whole genome oligo microarray 44k. Slides were scanned using an Agilent 2565 AB DNAmicroarray scanner. Microarray images were analysed by using Feature extraction software version A.8.5.1.1. (Agilent). Raw data files were then imported into Resolver® system for gene expression data analysis (Rosetta Inpharmatics LLC, Seattle, USA, WA).

### RT-QPCR

Reverse transcription and quantitative real time PCR were performed as described in (Brellier et al., 2008, Oncogene), using the specific primers (TABLE 6).

Results of the Q-PCR were normalized using the geNorm software (medgen.ugent.be/∼jvdesomp/genorm/). A Mann-Whitney test was used to determine whether the levels of mRNA in the 3 control and 6 NBCCS dermal equivalent were statistically different.

### ELISA

MMP1 and MMP3 levels in the supernatant of the dermal equivalents were measured using the human biotrak assays (RPN2610 and RPN 2613, GE Healthcare, London, UK) and FGF7 levels was determined using the Human KGF Immunoassay (DKG00, R&D Systems, Minneapolis, Minnesota), according to the manufacturers' protocols.

### Western blot

Proteic extracts were performed from classical 2D fibroblast cultures. Proteins (70 µg) were separated using a 12% SDS-PAGE, transferred onto polyvinyl difluoride membrane and probed with the rabbit polyclonal anti-SDF1 alpha (alias CXCL12) antibody (ab9797, Abcam, Cambridge, UK, 1/200). Membranes were then reprobed using the mouse monoclonal anti-GAPDH antibody (ab9484, Abcam,Cambridge, UK, 1/5000). Blots were revealed using electrochemiluminescence (ECL+) reagents (GE Healthcare, London, UK). Quantification was performed using genetools software (Ozyme, Montigny-le-Bretonneux, France). The levels of CXCL12 were normalized to the levels of glyceraldehyde 3 phosphate dehydrogenase (GAPDH).

**TABLE 1 : Common NBCCS signature of the microarray results: genes differentially expressed in NBCCS pools compared to control pool.**

| Primary Sequence Name | Accession Number | missense pool | | nonsense pool | |
|---|---|---|---|---|---|
| | | Fold Change | p-value | Fold Change | p-value |
| A_23_P170719 | | 6,1947 | 0 | 2,6836 | 3, 10E-24 |
| A_23_P255111 | | -1,7949 | 4,89E-14 | -5,2354 | 0 |
| A_24_P289854 | | 3,7382 | 7,02E-36 | 3,5528 | 3,95E-18 |
| A_24 P384210 | | 1,3293 | 8,66E-10 | 1,2797 | 1,77E-06 |
| A_24_P647682 | | 2,699 | 3,75E-26 | 2,6545 | 1,51E-15 |
| A_24_P752362 | | 2,9768 | 8,03E-23 | 2,8958 | 2,09E-34 |
| A_24_P934306 | | 2,4805 | 7,54E-15 | 2,6047 | 3,08E-11 |
| A_32_P75141 | | 6,1635 | 0 | 4,9618 | 0 |
| A2M | M_000014 | 8,6147 | 0 | 1,7405 | 2,44E-06 |
| AA417913 | AA417913 | 2,37 | 3,52E-19 | 2,409 | 1,49E-29 |
| AA516273 | AA516273 | 1,8088 | 1,28E-06 | 1,7599 | 3,24E-07 |
| ABCC3 | NM_003786 | 1,9821 | 1, 14E-09 | 1,6254 | 6,40E-08 |
| ACOT2 | NM_006821 | -1,5324 | 2,26E-11 | -1,4288 | 1,39E-06 |
| ADAM12 | M_003474 | -1,4238 | 2,64E-10 | -1,5128 | 4,89E-06 |
| ADARB1 | M_015833 | 2,2018 | 6,96E-29 | 2,4494 | 6,38E-38 |
| ADFP | M_001122 | 2,5132 | 4,54E-17 | 2,0442 | 4,12E-07 |
| ADH1A | NM_000667 | -1,3742 | 6,22E-06 | -1,3893 | 1,96E-08 |
| ADH4 | NM_000670 | -3,23 | 8,12E-12 | -3,827 | 4,91E-12 |
| AF155662 | AF155662 | 1,4373 | 1,26E-07 | 1,5008 | 4,04E-10 |
| AF264625 | AF264625 | 2,8355 | 7,77E-28 | 2,8518 | 2,37E-25 |
| AI476245 | AI476245 | 5,1206 | 0 | 3,0481 | 3,41E-17 |
| AJ276555 | AJ276555 | 2,0098 | 5,57E-16 | 2,046 | 4,57E-20 |
| AK026784 | AK026784 | -1,9424 | 1,36E-10 | -2,0477 | 5,27E-10 |
| AK055915 | AK055915 | -1,5952 | 4,73E-14 | -1,5486 | 1,08E-06 |
| AK075484 | AK075484 | -1,9695 | 9,44E-21 | -2,0015 | 6,05E-19 |
| AK126405 | AK126405 | -5,9166 | 0 | -2,7683 | 0 |
| AKR1C1 | M_001353 | 2,8523 | 5,59E-37 | 2,4218 | 7,18E-21 |
| AKR1C3 | NM_003739 | 2,3671 | 8,04E-21 | 2,3569 | 7,55E-24 |
| AL359052 | AL359052 | -2,0027 | 7,30E-16 | -2,0856 | 1,69E-13 |
| ALDH1A3 | NM_000693 | 5,92 | 0 | 10,883 | 0 |
| AMID | NM_032797 | 1,902 | 7,82E-21 | 1,5964 | 8,03E-07 |
| ANGPTL2 | NM_012098 | 2,0245 | 2,91E-14 | 1,6666 | 6,67E-08 |
| ANGPTL4 | NM_139314 | 7.5098 | 0 | 3.1122 | 0 |
| ANXA6 | NM_001155 | 1,8518 | 2,33E-17 | 1,6998 | 1,91E-09 |
| AOX1 | NM_001159 | 1,5893 | 1,80E-18 | 1,5901 | 3,26E-19 |
| APBB1IP | NM_019043 | 4,6804 | 0 | 3,7532 | 2,47E-40 |
| APOE | NM_000041 | -1,9402 | 1,11 E-42 | -1,3029 | 5,30E-08 |
| AQP1 | NM_000385 | -3,6666 | 0 | -2,5611 | 2,90E-36 |
| ARL6IP5 | NM_006407 | 1,3919 | 8,81 E-08 | 1,4065 | 1,34E-09 |
| ASNS | NM_001673 | -1,5371 | 3,94E-20 | -1,4911 | 1,58E-15 |
| ATP6V1B2 | NM_001693 | 2,045 | 8,66E-17 | 2,0845 | 6,81 E-15 |
| AW946823 | AW946823 | 2,5737 | 1,71E-14 | 2,0896 | 7,27E-08 |
| BASP1 | NM_006317 | 1,6281 | 4,33E-10 | 1,6043 | 4,29E-09 |
| BAX | NM_138764 | -1,4923 | 1,28E-07 | -1,3944 | 8,05E-07 |
| BC031278 | BC031278 | 1,7963 | 4,22E-22 | 1,7613 | 1,68E-26 |
| BC035260 | BC035260 | 2,1041 | 3,96E-12 | 2,4093 | 6,50E-09 |
| BC037430 | BC037430 | -2,2074 | 4,78E-30 | -1,9038 | 7,93E-18 |
| BC062473 | BC062473 | 2,5805 | 4,85E-40 | 1,7842 | 1,50E-20 |
| BC066344 | BC066344 | -1,7401 | 2,21E-18 | -1,7629 | 3,65E-10 |
| BF213738 | BF213738 | -1,7398 | 4,65E-19 | -1,6952 | 4,92E-32 |
| BM921275 | BM921275 | 1,3403 | 3,58E-06 | 1,3433 | 2,70E-07 |
| BNIP3 | NM_004052 | 1,8772 | 2,21 E-27 | 1,411 | 6,29E-09 |
| BST2 | NM_004335 | -3,2752 | 0 | -3,0303 | 1,29E-15 |
| BTG1 | NM_001731 | 1,5781 | 7,71E-08 | 1,6958 | 4,54E-07 |
| BX537732 | BX537732 | 1,8139 | 2,50E-13 | 1,6883 | 1,52E-10 |
| BX537788 | BX537788 | 4,3842 | 9,63E-37 | 3,408 | 1,22E-15 |
| C14orf147 | NM_138288 | -1,5243 | 1,08E-09 | -1,4347 | 4,55E-10 |
| C1GALT1C1 | NM_152692 | 1,7104 | 7,56E-18 | 1,4669 | 1,64E-09 |
| C1orf188 | NM_173795 | -1,2827 | 8,52E-09 | -1,2649 | 2,08E-07 |
| C1orf42 | NM_019060 | 1,7216 | 1,16E-12 | 1,6265 | 1,08E-09 |
| C20orf19 | NM_018474 | 1,6635 | 1,96E-12 | 1,5786 | 1,30E-10 |
| C6orf48 | NM_016947 | -1,317 | 9,10E-08 | -1,2647 | 2,96E-07 |
| C9orf150 | NM_203403 | -1,4285 | 7,33E-09 | -1,6064 | 1,34E-07 |
| CA12 | NM_001218 | 2,9836 | 0 | 2,1333 | 0 |
| CARHSP1 | NM_014316 | 1,5807 | 7,01E-06 | 1,8301 | 3,00E-13 |
| CCRL1 | NM_178445 | 2,3757 | 1,12E-18 | 2,4162 | 1,81E-26 |
| CD44 | NM_000610 | 1,7313 | 1,91E-12 | 1,5131 | 5,27E-06 |
| CD99 | NM_002414 | 1,4622 | 3,18E-09 | 1,3388 | 7,41E-10 |
| CDC42EP3 | NM_006449 | -1,5728 | 1,15E-07 | -1,5648 | 1,42E-10 |
| CDH13 | NM_001257 | 1,611 | 7,47E-07 | 2,1522 | 2,60E-15 |
| CDON | AK022986 | 2,6989 | 2,02E-19 | 2,2717 | 1,60E-08 |
| CH25H | NM_003956 | -2,062 | 0 | -1,6382 | 4,24E-14 |
| CHN1 | NM_001822 | -2,0453 | 1,34E-16 | -2,1094 | 4,55E-20 |
| CHST2 | NM_004267 | -1,4066 | 4,42E-07 | -1,6757 | 4,72E-08 |
| CHST7 | NM_019886 | -1,9272 | 2.91E-26 | -2,4096 | 4.61E-28 |
| CILP | NM_003613 | -1,9239 | 2,27E-09 | 1,9266 | 3,45E-08 |
| CLEC2B | NM_005127 | -1,6982 | 8,23E-26 | -1,6084 | 8,22E-16 |
| CLEC3B | NM_003278 | -2,016 | 0 | 1,3543 | 2,05E-13 |
| CLIC6 | NM_053277 | 18,5676 | 1,72E-35 | 7,711 | 3,54E-13 |
| CMTM1 | NM_181289 | 2,3129 | 3,71E-31 | 2,3448 | 1,11E-25 |
| COL11A1 | NM_080629 | 9.7967 | 9,06E-22 | 5.4611 | 4,28E-14 |
| COL12A1 | NM_004370 | 2,3825 | 9,44E-37 | 1,6513 | 4,51E-10 |
| COL1A1 | NM_000088 | -1,2561 | 8,93E-07 | -1,4463 | 6,55E-13 |
| COL1A2 | NM_000089 | -1,4806 | 9,14E-12 | -1,3149 | 8,51E-08 |
| COL23A1 | NM_173465 | -3,6652 | 7,01E-45 | -2,1148 | 4,82E-14 |
| COL3A1 | NM_000090 | -1,4326 | 6,65E-09 | -1,5389 | 5,69E-12 |
| COL5A1 | NM_000093 | -1,4651 | 1,11 E-13 | -1,7266 | 2,36E-26 |
| COL7A1 | NM_000094 | -1,8471 | 2,77E-21 | -1,347 | 7,06E-05 |
| COLEC12 | NM_030781 | 1,275 | 9,63E-07 | 1,8187 | 7,71E-17 |
| COPS2 | NM_004236 | 1,4181 | 2,95E-06 | 1,4172 | 1,64E-07 |
| CPM | NM_001874 | 3,715 | 0 | 2,1577 | 1,14E-25 |
| CPT1C | NM_152359 | -2,1895 | 5,27E-42 | -1,4124 | 8,16E-06 |
| CPXM2 | NM_198148 | -5,9272 | 2,66E-44 | -2,223 | 7,27E-09 |
| CR617391 | CR617391 | -1,4559 | 4,50E-09 | -1,8749 | 8,05E-27 |
| CSEN | NM_013434 | -3,072 | 0 | -2,0124 | 1,27E-29 |
| CSPG4 | NM_001897 | -2,3191 | 2,53E-16 | -1,9602 | 4,89E-07 |
| CSRP2 | NM_001321 | -2,3663 | 1,94E-30 | -1,6951 | 2,09E-13 |
| CTHRC1 | NM_138455 | 2,5446 | 5,94E-20 | 1,6947 | 4,59E-07 |
| CTSB | AK097384 | 1,2593 | 3,13E-07 | 1,4216 | 6,47E-08 |
| CUGBP2 | NM_006561 | 1,8895 | 7,44E-24 | 1,8691 | 2,36E-28 |
| CXCL1 | NM_001511 | -1,7183 | 3,77E-07 | -2,1977 | 6,06E-12 |
| CXCL12 | NM_199168 | 2.8062 | 1,10E-34 | 2.5629 | 5,33E-25 |
| CXCL2 | NM_002089 | -1,4518 | 3,83E-08 | -2,0825 | 1,99E-08 |
| CYP1B1 | NM_000104 | 1,3155 | 1,74E-10 | -1,4357 | 6,11E-16 |
| CYR61 | NM_001554 | 1,5646 | 9,26E-24 | 1,2641 | 2,19E-08 |
| DHRS3 | NM_004753 | 4,3342 | 0 | 3,7553 | 3,85E-33 |
| DIRC1 | NM_052952 | 1,5025 | 6,06E-20 | 1,421 | 4,71E-09 |
| DKFZp434L142 | NM_016613 | -2,2053 | 0 | -1,8556 | 1,41E-22 |
| DKK1 | NM_012242 | 3,3686 | 0 | 1,6358 | 3,03E-26 |
| DKK3 | NM_013253 | -3,3895 | 6,82E-26 | -1,9717 | 3,39E-10 |
| DLST | NM_001933 | 1,377 | 9,10E-13 | 1,4567 | 7,80E-15 |
| DPP3 | NM_130443 | 2,6339 | 8,64E-18 | 2,6929 | 3,39E-17 |
| DPYSL4 | NM_006426 | 1,7711 | 6,96E-10 | 1,61 | 1,01E-07 |
| DSCR1 | NM_004414 | 2,2516 | 1,42E-10 | 2,4037 | 1,24E-11 |
| DSCR1L1 | NM_005822 | -9,6226 | 0 | -3,713 | 0 |
| E2IG5 | NM_014367 | 1,7059 | 6,19E-18 | 1,3819 | 1,00E-07 |
| ECHDC3 | NM_024693 | -4,6909 | 1,88E-23 | -5,9668 | 1,37E-24 |
| EDARADD | NM_145861 | 1,4836 | 7,08E-11 | 1,339 | 1,16E-10 |
| EFEMP1 | NM_004105 | 2,5939 | 0 | 1,5247 | 1,01E-10 |
| EMILIN2 | NM_032048 | 1,4158 | 1,68E-13 | 1,4087 | 1,12E-13 |
| EMP2 | NM_001424 | 1,9756 | 4,75E-20 | 1,7299 | 2,52E-22 |
| EMX2 | NM_004098 | 1,5242 | 5,28E-06 | 1,7484 | 1,71E-08 |
| EMX2OS | AY117034 | 1,6578 | 5,59E-11 | 1,8749 | 5,76E-16 |
| ENST00000296496 | CR597270 | 1,472 | 2,94E-08 | 1,2854 | 7,44E-06 |
| EVC | NM_014556 | -2,5052 | 1,34E-12 | -1,6697 | 7,79E-08 |
| EXOSC6 | NM_058219 | 2,2792 | 0 | 2,3187 | 1,12E-44 |
| EYA2 | NM_172113 | -4,8738 | 0 | -1,901 | 7,22E-10 |
| FBLN2 | NM_001998 | -1,259 | 9,61E-08 | -1,2602 | 4,53E-06 |
| FBN1 | NM_000138 | -1,3008 | 5,64E-09 | -1,3062 | 1,59E-06 |
| FDXR | NM_004110 | -1,4703 | 1,18E-06 | -1,3251 | 4,18E-09 |
| FGF7 | NM_002009 | 2,3111 | 8,39E-30 | 1,5902 | 2,05E-18 |
| FKBP10 | NM_021939 | 1,4919 | 3,32E-11 | 1,4244 | 6,35E-11 |
| FLJ10157 | AK001019 | -2,5374 | 3,10E-10 | -3,7488 | 1,34E-18 |
| FLJ11125 | AK001987 | 1,3603 | 6,21E-08 | 1,3274 | 2,94E-11 |
| FLJ14834 | NM_032849 | 5,8414 | 0 | 2,7641 | 6,60E-32 |
| FLJ30435 | NM_174950 | 2,284 | 6,79E-22 | 1,4957 | 5,59E-07 |
| FMOD | NM_002023 | 3,1081 | 0 | 1,8023 | 2,31E-16 |
| FPR1 | NM_002029 | -1,741 | 9,19E-20 | -1,6412 | 2,19E-09 |
| FST | NM_013409 | -1,5995 | 1,22E-17 | -1,5192 | 1,61 E-09 |
| GALNTL2 | NM_054110 | 2,0129 | 9,76E-41 | 1,5649 | 8,93E-09 |
| GHR | NM_000163 | 4,5814 | 9,60E-26 | 3,0934 | 4,03E-06 |
| GLRX | NM_002064 | 1,5039 | 8,74E-20 | 1,3365 | 1,71E-08 |
| GLT8D2 | NM_031302 | -1,5234 | 1,62E-09 | -1,5828 | 2,18E-06 |
| GPC6 | BX640888 | 3,4137 | 7,47E-36 | 2,405 | 7,60E-21 |
| GPT | NM_005309 | 1,5459 | 3,93E-25 | 1,5546 | 3,00E-16 |
| GRB10 | NM_001001555 | -1,4508 | 3,02E-16 | -1,3402 | 1,81E-06 |
| GREM1 | NM_013372 | 1,8648 | 4,03E-14 | 1,7318 | 4,64E-15 |
| HIG2 | NM_013332 | 1,8775 | 1,12E-44 | 1,4082 | 1,38E-13 |
| HSPA5BP1 | NM_017870 | 2,8592 | 5,23E-18 | 2,2717 | 4,12E-15 |
| HSPB2 | NM_001541 | 1,5429 | 1,13E-08 | 1,43 | 3,40E-07 |
| HTRA1 | NM_002775 | -1,2794 | 7,07E-06 | -1,3294 | 1,33E-09 |
| ID2 | NM_002166 | 2,2745 | 7,96E-41 | 1,6013 | 7,44E-17 |
| IFI27 | NM_005532 | -4,1996 | 0 | -4,7571 | 0 |
| IGFBP2 | NM_000597 | -2,1809 | 7,33E-30 | -4,2923 | 0 |
| IGFBP7 | NM_001553 | -1,891 | 2,89E-06 | -6,0029 | 0 |
| IL13RA2 | NM_000640 | 8,9084 | 0 | 4,6296 | 2,00E-19 |
| IL15 | NM_172174 | -1,3507 | 2,18E-10 | -1,3858 | 1,92E-06 |
| IL27 | NM_145659 | 1,4436 | 1,07E-13 | 1,3817 | 2,70E-11 |
| INHBB | NM_002193 | -1,5886 | 8,37E-26 | -1,5278 | 1,13E-16 |
| ITGB5 | NM_002213 | 1,9381 | 2,75E-17 | 1,5957 | 4,74E-07 |
| ITGBL1 | NM_004791 | -1,716 | 9,16E-19 | -1,9716 | 1,56E-27 |
| KCNJ8 | NM_004982 | 12,2448 | 0 | 10,1035 | 5,14E-32 |
| KCTD15 | NM_024076 | 3,0865 | 1,44E-21 | 3,0498 | 1,42E-19 |
| KIAA0664 | NM_015229 | 1,857 | 1,91E-31 | 1,8454 | 3,04E-32 |
| KIAA1199 | NM_018689 | 1,4213 | 8,36E-09 | 1,6891 | 4,35E-12 |
| KLF6 | NM_001300 | 1,5373 | 4,44E-23 | 1,3389 | 9,78E-10 |
| KRTAP5-8 | NM_021046 | 1,522 | 3,46E-13 | 1,4603 | 6,31E-09 |
| LAMA2 | NM_000426 | -1,5328 | 2,11 E-09 | -1,4533 | 7,55E-09 |
| LAYN | NM_178834 | -1,4764 | 4,06E-07 | -1,4906 | 3,51E-13 |
| LCE2A | NM_178428 | 2,1528 | 2,54E-35 | 2,0718 | 2,23E-17 |
| LEPROT | NM_017526 | 1,727 | 7,73E-16 | -1,5699 | 5,37E-11 |
| LFNG | BC014851 | -1,9288 | 4,82E-12 | -1,6955 | 1,12E-06 |
| LMOD1 | NM_012134 | 1,709 | 8,39E-10 | 1,7694 | 8,53E-10 |
| LOC375295 | BC013438 | -2,4275 | 3,41E-20 | -2,7879 | 1,25E-14 |
| LOC387763 | BC052560 | 2,6507 | 3,76E-34 | 1,7848 | 1,72E-07 |
| LOC388610 | BC069216 | -1,3732 | 2,17E-07 | -1,3316 | 1,49E-10 |
| LOC389652 | XM_372040 | -1,5413 | 1,23E-11 | -1,5574 | 1,76E-15 |
| LOC441245 | AK090474 | 1,4213 | 3,06E-10 | 1,3605 | 3,93E-10 |
| LOC442293 | XM_498178 | -1,6856 | 2,53E-10 | -1,6404 | 1,30E-07 |
| LOXL3 | NM_032603 | -1,4986 | 1,39E-07 | -2,0143 | 9,42E-27 |
| LRAP | NM_022350 | -1,5236 | 1,47E-07 | -2,1474 | 2,18E-09 |
| LRIG3 | NM_153377 | -1,6875 | 1,65E-17 | -1,4743 | 4,33E-10 |
| LUM | NM_002345 | -2,1416 | 0 | -2,1803 | 3,21E-31 |
| LY6K | NM_017527 | -1,3724 | 1,36E-12 | 1,4197 | 7,08E-10 |
| MAB21L2 | NM_006439 | 1,888 | 3,08E-13 | 1,8594 | 2,71E-20 |
| MALAT1 | BX538238 | -1,5079 | 9,29E-15 | -1,45 | 1,11E-12 |
| MARVELD1 | NM_031484 | -1,5971 | 8,69E-11 | -1,6071 | 1,12E-09 |
| MASP1 | NM_139125 | 3,2252 | 1,34E-21 | 3,421 | 3,33E-18 |
| MGC42157 | BC030111 | 2,9857 | 0 | 2,759 | 1,59E-22 |
| MGP | NM_000900 | 2,0439 | 1,75E-14 | 1,4461 | 1,58E-07 |
| MGST1 | NM_145791 | 3,9293 | 0 | 2,5332 | 2,55E-24 |
| MLPH | NM_024101 | -1,9409 | 1,83E-16 | -1,7616 | 1,33E-09 |
| MMP1 | NM_002421 | 8.4122 | 0 | 7.8028 | 0 |
| MMP3 | NM_002422 | 19.7421 | 0 | 13.0139 | 0 |
| MRPS6 | NM_032476 | -2,5203 | 1,31E-25 | -2,004 | 6,07E-23 |
| MT | NM_014507 | 1,4584 | 5,18E-20 | 1,3918 | 6,95E-11 |
| MUC20 | NM_152673 | -1,936 | 1,15E-06 | -2,4596 | 1,41E-21 |
| MYLK | NM_053025 | -2,2814 | 1,44E-16 | -2,2724 | 1,14E-17 |
| NANOS1 | NM_199461 | 3,609 | 3,75E-13 | 2,6956 | 5,74E-12 |
| NDN | NM_002487 | 1,5226 | 2,98E-08 | 1,5537 | 1,53E-09 |
| NDUFA12 | NM_018838 | -1,4427 | 1,91E-10 | -1,3934 | 2,95E-08 |
| NET1 | NM_005863 | -2,1241 | 3,03E-31 | -1,4669 | 1,45E-08 |
| NOPE | NM_020962 | -2,2406 | 6,31E-09 | -2,1367 | 1,82E-07 |
| NR4A3 | NM_173200 | 4,2339 | 0 | 2,1442 | 3,44E-11 |
| NT5E | NM_002526 | 1,6194 | 1,80E-08 | 1,7122 | 1,21E-10 |
| PAM | NM_000919 | 1,4992 | 1,03E-11 | 1,497 | 1,55E-09 |
| PCSK5 | NM_006200 | 3,4846 | 0 | 2,1278 | 1,36E-09 |
| PDGFRB | NM_002609 | -1,6261 | 4,06E-20 | -1,4109 | 1,15E-12 |
| PDIA3 | NM_005313 | -1,5006 | 4,06E-08 | -1,5565 | 1,59E-07 |
| PDPN | NM_006474 | 1,5016 | 6,68E-07 | 1,9428 | 1,23E-26 |
| PGM1 | NM_002633 | 1,9421 | 9,20E-14 | 1,9195 | 9,82E-17 |
| PHLDA1 | NM_007350 | -1,6221 | 6,42E-24 | -1,5427 | 1,52E-14 |
| PITPNC1 | NM_181671 | 2,0932 | 4,25E-08 | 2,0012 | 3,73E-06 |
| PLA2G4A | NM_024420 | 1,3564 | 2,86E-07 | 2,2496 | 0 |
| PLS3 | NM_005032 | 2,1629 | 2,04E-41 | 1,3926 | 2,64E-09 |
| pp9099 | NM_025201 | -2,2115 | 1,39E-20 | -1,5228 | 1,59E-07 |
| PPAP2A | NM_176895 | 3,5108 | 6,87E-38 | 2,23 | 1,83E-35 |
| PRL | NM_000948 | 5,4189 | 0 | 2,484 | 2,42E-15 |
| PRNP | NM_000311 | -1,3309 | 5,69E-12 | -1,3382 | 1,51E-10 |
| PRSS36 | NM_173502 | 1,4489 | 9,07E-11 | 1,4285 | 2,60E-14 |
| PSAT1 | NM_058179 | -1,7732 | 1,28E-18 | -1,825 | 4,65E-21 |
| PTGDS | NM_000954 | -3,9356 | 0 | -1,9318 | 9,78E-25 |
| PTGIS | NM_000961 | 2,8252 | 5,37E-22 | 2,7335 | 2,82E-23 |
| PTPRK | NM_002844 | -1,5308 | 8,24E-12 | -1,3486 | 3,46E-06 |
| PYCR1 | NM_153824 | -1,2966 | 6,64E-06 | -1,3304 | 3,66E-08 |
| QPCT | NM_012413 | 2,1039 | 8,56E-24 | 1,3834 | 3,07E-07 |
| RAB31 | NM_006868 | -1,2302 | 8,20E-06 | -1,4304 | 1,88E-09 |
| RECK | NM_021111 | 1,508 | 1,12E-06 | 1,455 | 2,05E-07 |
| REV3L | NM_002912 | 1,4361 | 1,75E-06 | 1,5779 | 6,18E-07 |
| RPLP0 | BF570115 | 1,4295 | 1,79E-09 | 1,593 | 1,02E-09 |
| RSPO3 | NM_032784 | -2,2446 | 4,25E-10 | -2,7686 | 1,58E-14 |
| RUTBC3 | NM_015705 | -1,6092 | 3,51E-06 | -1,626 | 4,81E-07 |
| SAA1 | NM_000331 | 3,048 | 0 | 2,1112 | 1,25E-21 |
| SAA2 | NM_030754 | 2,4569 | 0 | 1,9001 | 4,05E-15 |
| SAMD10 | NM_080621 | 2,0178 | 6,03E-27 | 1,9777 | 9,01E-38 |
| SAT | NM_002970 | 1,9729 | 1,26E-21 | 1,7193 | 3,93E-10 |
| SCARB1 | NM_005505 | -1,6479 | 3,66E-21 | -1,6919 | 8,39E-15 |
| SERP1 | NM_014445 | -1,3356 | 5,69E-08 | -1,3277 | 3,49E-06 |
| SERPINF1 | NM_002615 | -2,0094 | 9,40E-09 | -1,6694 | 3,12E-06 |
| SFRP2 | NM_003013 | 1,9534 | 4,84E-28 | 2,5841 | 3,79E-33 |
| SFRS5 | NM_006925 | -2,334 | 1,06E-37 | -2,1145 | 1,68E-42 |
| SGCE | NM_003919 | 1,4298 | 9,05E-08 | 1,4387 | 8,35E-06 |
| SLC20A1 | NM_005415 | -1,3303 | 3,16E-11 | -1,2623 | 1,80E-06 |
| SLC39A8 | NM_022154 | 2,6101 | 3,18E-11 | 2,0025 | 9,21E-07 |
| SMAD3 | NM_005902 | 2,2706 | 2,19E-25 | 1,8951 | 4,67E-18 |
| SMOC2 | NM_022138 | 5,5702 | 6,90E-20 | 2,788 | 4,85E-06 |
| SMPDL3A | NM_006714 | 1,5995 | 1,41E-15 | 1,396 | 6,76E-07 |
| SNCA | NM_007308 | 3,0297 | 3,71E-30 | 2,5018 | 7,41 E-09 |
| SOD2 | NM_000636 | 1,6025 | 1,15E-19 | 1,7405 | 1,10E-20 |
| SOX4 | NM_003107 | 1,8556 | 9,70E-14 | 1,6116 | 3,56E-08 |
| SPOCK1 | NM_004598 | 3,1251 | 0 | 3,3341 | 0 |
| SPON2 | NM_012445 | -4,953 | 0 | -1,8769 | 5,91E-13 |
| SPSB2 | NM_032641 | -2,1485 | 1,91E-12 | -2,1705 | 1,94E-14 |
| STEAP1 | NM_012449 | 2,9091 | 0 | 2,3808 | 7,46E-35 |
| STEAP2 | NM_152999 | 2,8101 | 7,94E-37 | 2,2325 | 1,10E-09 |
| SVEP1 | AK075235 | 1,5754 | 9,36E-13 | 1,5834 | 9,27E-17 |
| SYNPO | AB028952 | 1,9568 | 4,59E-14 | 2,0189 | 3,50E-21 |
| TBX1 | NM_080647 | -5,1205 | 0 | -4,7772 | 0 |
| TBX5 | NM_000192 | -3,1129 | 3,38E-12 | -3,536 | 1,15E-07 |
| TFPI2 | NM_006528 | 5,533 | 0 | 2,0953 | 5,21 E-21 |
| THBD | NM_000361 | 1,8919 | 1,34E-08 | 1,9982 | 1,26E-07 |
| THC2042343 | | -1,437 | 5,90E-12 | -1,2269 | 4,42E-06 |
| THC2051764 | | -1,4189 | 3,56E-07 | -1,4977 | 6,31 E-09 |
| THC2054763 | | -1,5113 | 2,97E-09 | -1,8521 | 2,73E-06 |
| THC2088849 | | 1,3662 | 5,54E-11 | 1,4553 | 5,50E-12 |
| THC2100006 | | 1,6642 | 6,75E-12 | 1,6337 | 4,60E-09 |
| THC2104265 | AI358607 | 1,8521 | 1,36E-20 | 1,7994 | 1,51E-29 |
| THC2105523 | | -7,1402 | 0 | -5,2897 | 0 |
| THC2140046 | BG428517 | 2,927 | 0 | 2,0764 | 1,36E-24 |
| THC2173240 | | -2,0246 | 2,89E-09 | -1,6599 | 2,17E-10 |
| THC2208133 | AI620901 | -2,0711 | 2,25E-11 | -1,9838 | 9,53E-08 |
| THC2230875 | | 1,5103 | 2,79E-13 | 1,4767 | 8,09E-13 |
| TNC | NM_002160 | 1,4002 | 1,67E-10 | 1,3454 | 2,12E-06 |
| TncRNA | AK027191 | -1,6019 | 7,49E-11 | -1,4575 | 1,98E-13 |
| TNFAIP2 | NM_006291 | 1,4815 | 2,03E-18 | 1,4017 | 3,86E-08 |
| TNFRSF19 | NM_148957 | -6,1012 | 6,96E-32 | -3,8355 | 9,09E-15 |
| TNXB | NM_019105 | 1,5661 | 3,94E-07 | 2,8929 | 8,75E-36 |
| TPCN1 | AK000619 | 1,2438 | 1,53E-06 | 1,6269 | 8,66E-15 |
| TRPV2 | NM_016113 | -2,258 | 0 | -1,2706 | 9,04E-06 |
| TUBA1 | NM_006000 | 2,3766 | 3,08E-27 | 2,0994 | 7,09E-10 |
| UCHL1 | NM_004181 | 2,2284 | 0 | 1,7156 | 1,55E-25 |
| WIPI1 | NM_017983 | -1,4296 | 2,05E-09 | -1,3844 | 3,39E-07 |
| WISP2 | NM_003881 | 2.5103 | 1,30E-23 | 2.1266 | 6,04E-18 |
| WNT5A | NM_003392 | -2,9324 | 0 | -2,7149 | 0 |
| X68990 | X68990 | 1,5084 | 6,73E-16 | 1,4834 | 1,07E-16 |
| ZBED3 | NM_032367 | 1,4 | 2,62E-07 | 1,4781 | 6,17E-06 |
| ZFP36L2 | NM_006887 | 1,4351 | 3,82E-16 | 1,4675 | 2,11E-17 |
| ZNF179 | NM_007148 | -2,5971 | 1,42E-25 | -1,7311 | 1,70E-06 |

**TABLE 2 : Anti-correlated genes between the missense and the nonsense pools.**

| Primary Sequence Name | Accession Number | missense pool | | nonsense pool | |
|---|---|---|---|---|---|
| | | Fold Change | p-value | Fold Change | p-value |
| CILP | M_003613 | -1,9239 | 2,27E-09 | 1,9266 | 3,45E-08 |
| LY6K | NM_017527 | -1,3724 | 1,36E-12 | 1,4197 | 7,08E-10 |
| LY6K | NM_017527 | -1,4 | 5,68E-08 | 1,3563 | 8,97E-12 |
| CLEC3B | NM_003278 | -2,016 | 0 | 1,3543 | 2,05E-13 |
| CYP1B1 | NM_000104 | 1,3155 | 1,74E-10 | -1,4357 | 6,11E-16 |
| LEPROT | NM_017526 | 1,727 | 7,73E-16 | -1,5699 | 5,37E-11 |

**TABLE 3 : Narrow NBCCS signature mostly comprising more than two fold up-regulated genes in the two NBCCS pools and significant down-regulated genes**

| Primary Sequence Name | Accession Number | missense pool | | nonsense pool | |
|---|---|---|---|---|---|
| | | Fold Increase | p-value | Fold Increase | p-value |
| A_23_P170719 | | 6.1947 | 0 | 2.6836 | 3,10E-24 |
| A_24_P289854 | | 3.7382 | 7,02E-36 | 3.5528 | 3,95E-18 |
| A_24_P647682 | | 2.699 | 3,75E-26 | 2.6545 | 1,51E-15 |
| A_24_P752362 | | 2.9768 | 8,03E-23 | 2.8958 | 2,09E-34 |
| A_24_P934306 | | 2.4805 | 7,54E-15 | 2.6047 | 3,08E-11 |
| A_32_P75141 | | 6.1635 | 0 | 4.9618 | 0 |
| AA417913 | AA417913 | 2.37 | 3,52E-19 | 2.409 | 1,49E-29 |
| ADARB1 | NM_015833 | 2.2018 | 6,96E-29 | 2.4494 | 6,38E-38 |
| ADFP | NM_001122 | 2.5132 | 4,54E-17 | 2.0442 | 4,12E-07 |
| AF264625 | AF264625 | 2.8355 | 7,77E-28 | 2.8518 | 2,37E-25 |
| AI476245 | AI476245 | 5.1206 | 0 | 3.0481 | 3,41E-17 |
| AJ276555 | AJ276555 | 2.0098 | 5,57E-16 | 2.046 | 4,57E-20 |
| AKR1C1 | NM_001353 | 2.8523 | 5,59E-37 | 2.4218 | 7,18E-21 |
| AKR1C3 | NM_003739 | 2.3671 | 8,04E-21 | 2.3569 | 7,55E-24 |
| ALDH1A3 | NM_000693 | 5.92 | 0 | 10.883 | 0 |
| ANGPTL2 | NM_012098 | 2,0245 | 2,91E-14 | 1,6666 | 6,67E-08 |
| ANGPTL4 | NM_139314 | 7.5098 | 0 | 3.1122 | 0 |
| APBB1IP | NM_019043 | 4.6804 | 0 | 3.7532 | 2,47E-40 |
| ATP6V1B2 | NM_001693 | 2.045 | 8,66E-17 | 2.0845 | 6,81 E-15 |
| AW946823 | AW946823 | 2.5737 | 1,71E-14 | 2.0896 | 7,27E-08 |
| BC035260 | BC035260 | 2.1041 | 3,96E-12 | 2.4093 | 6,50E-09 |
| BX537788 | BX537788 | 4.3842 | 9,63E-37 | 3.408 | 1,22E-15 |
| CA12 | NM_001218 | 2.9836 | 0 | 2.1333 | 0 |
| CCRL1 | NM_178445 | 2.3757 | 1,12E-18 | 2.4162 | 1,81E-26 |
| CDON | AK022986 | 2.6989 | 2,02E-19 | 2.2717 | 1,60E-08 |
| CLIC6 | NM_053277 | 18.5676 | 1.72E-35 | 7.711 | 3,54E-13 |
| CMTM1 | NM_181289 | 2.3129 | 3.71E-31 | 2.3448 | 1,11E-25 |
| COL11A1 | NM_080629 | 9.7967 | 9,06E-22 | 5.4611 | 4,28E-14 |
| COL3A1 | NM_000090 | -1,4326 | 6,65E-09 | -1,5389 | 5,69E-12 |
| COL7A1 | NM_000094 | -1,8471 | 2,77E-21 | -1,347 | 7,06E-05 |
| CPM | NM_001874 | 3.715 | 0 | 2.1577 | 1,14E-25 |
| CXCL12 | NM_199168 | 2.8062 | 1,10E-34 | 2.5629 | 5,33E-25 |
| DHRS3 | NM_004753 | 4.3342 | 0 | 3.7553 | 3,85E-33 |
| DKK3 | NM_013253 | -3,3895 | 6,82E-26 | -1,9717 | 3,39E-10 |
| DPP3 | NM_130443 | 2.6339 | 8,64E-18 | 2.6929 | 3,39E-17 |
| DSCR1 | NM_004414 | 2.2516 | 1,42E-10 | 2.4037 | 1,24E-11 |
| EXOSC6 | NM_058219 | 2.2792 | 0 | 2.3187 | 1,12E-44 |
| FGF7 | NM_002009 | 2,3111 | 8,39E-30 | 1,5902 | 2,05E-18 |
| FLJ14834 | NM_032849 | 5.8414 | 0 | 2.7641 | 6,60E-32 |
| GHR | NM_000163 | 4.5814 | 9,60E-26 | 3.0934 | 4,03E-06 |
| GPC6 | BX640888 | 3.4137 | 7,47E-36 | 2.405 | 7,60E-21 |
| GREM1 | NM_013372 | 1,8648 | 4,03E-14 | 1,7318 | 4,64E-15 |
| HSPA5BP1 | NM_017870 | 2.8592 | 5,23E-18 | 2.2717 | 4,12E-15 |
| ID2 | NM_002166 | 2,2745 | 7,96E-41 | 1,6013 | 7,44E-17 |
| IL13RA2 | NM_000640 | 8.9084 | 0 | 4.6296 | 2,00E-19 |
| KCNJ8 | NM_004982 | 12.2448 | 0 | 10.1035 | 5,14E-32 |
| KCTD15 | NM_024076 | 3.0865 | 1,44E-21 | 3.0498 | 1,42E-19 |
| LAMA2 | NM_000426 | -1,5328 | 2,11E-09 | -1,4533 | 7,55E-09 |
| LCE2A | NM_178428 | 2.1528 | 2,54E-35 | 2.0718 | 2,23E-17 |
| MASP1 | NM_139125 | 3.2252 | 1,34E-21 | 3.421 | 3,33E-18 |
| MGC42157 | BC030111 | 2.9857 | 0 | 2.759 | 1,59E-22 |
| MGP | NM_000900 | 2,0439 | 1,75E-14 | 1,4461 | 1,58E-07 |
| MGST1 | NM_145791 | 3.9293 | 0 | 2.5332 | 2,55E-24 |
| MMP1 | NM_002421 | 8.4122 | 0 | 7.8028 | 0 |
| MMP3 | NM_002422 | 19.7421 | 0 | 13.0139 | 0 |
| NANOS1 | NM_199461 | 3.609 | 3,75E-13 | 2.6956 | 5,74E-12 |
| NR4A3 | NM_173200 | 4.2339 | 0 | 2.1442 | 3,44E-11 |
| PCSK5 | NM_006200 | 3.4846 | 0 | 2.1278 | 1,36E-09 |
| PITPNC1 | NM_181671 | 2.0932 | 4,25E-08 | 2.0012 | 3,73E-06 |
| PPAP2A | NM_176895 | 3.5108 | 6,87E-38 | 2.23 | 1,83E-35 |
| PRL | NM_000948 | 5.4189 | 0 | 2.484 | 2,42E-15 |
| PTGIS | NM_000961 | 2.8252 | 5,37E-22 | 2.7335 | 2,82E-23 |
| SAA1 | NM_000331 | 3.048 | 0 | 2.1112 | 1,25E-21 |
| SFRP2 | NM_003013 | 1,9534 | 4,84E-28 | 2,5841 | 3,79E-33 |
| SLC39A8 | NM_022154 | 2.6101 | 3,18E-11 | 2.0025 | 9,21 E-07 |
| SMOC2 | NM_022138 | 5.5702 | 6,90E-20 | 2.788 | 4,85E-06 |
| SNCA | NM_007308 | 3.0297 | 3,71 E-30 | 2.5018 | 7,41 E-09 |
| SPOCK1 | NM_004598 | 3.1251 | 0 | 3.3341 | 0 |
| STEAP1 | NM_012449 | 2.9091 | 0 | 2.3808 | 7,46E-35 |
| STEAP2 | NM_152999 | 2.8101 | 7,94E-37 | 2.2325 | 1,10E-09 |
| TFPI2 | NM_006528 | 5.533 | 0 | 2.0953 | 5,21 E-21 |
| THC2140046 | BG428517 | 2.927 | 0 | 2.0764 | 1,36E-24 |
| TNC | NM_002160 | 1,4002 | 1,67E-10 | 1,3454 | 2,12E-06 |
| TUBA1 | NM_006000 | 2.3766 | 3,08E-27 | 2.0994 | 7,09E-10 |
| WISP2 | NM_003881 | 2.5103 | 1,30E-23 | 2.1266 | 6,04E-18 |
| WNT5A | NM_003392 | -2,9324 | 0 | -2,7149 | 0 |

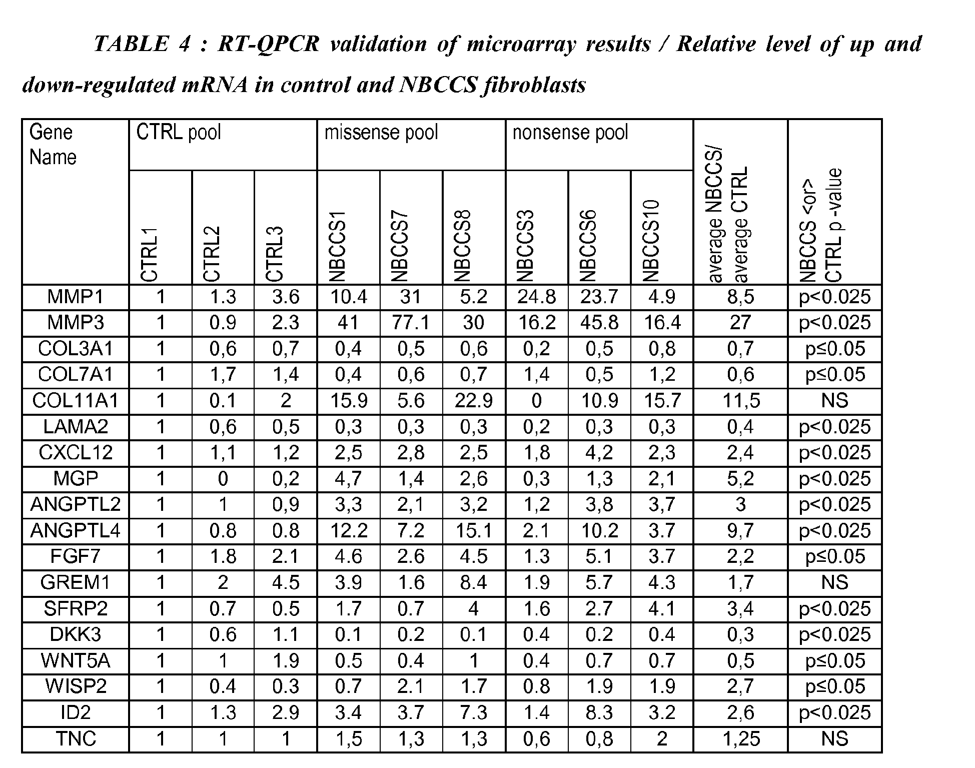

**TABLE 5 : Preferred NBCCS signature**

| Gene | Accession Number | Sequence Description |
|---|---|---|
| ANGPTL2 | NM_012098 | Angiopoietin-like 2 |
| ANGPTL4 | NM_139314 | Angiopoietin-like 4 |
| COL11A1 | NM_080629 | Collagen, type XI, alpha 1 |
| COL3A1 | NM_000090 | Collagen, type III, alpha 1 |
| COL7A1 | NM_000094 | Collagen, type VII, alpha 1 |
| CXCL12 | NM_199168 | Chemokine (C-X-C motif) ligand 12 |
| DKK3 | NM_013253 | Dickkopf homolog 3 |
| FGF7 | NM_002009 | Fibroblast growth factor 7 |
| GREM1 | NM_013372 | Gremlin 1, cysteine knot superfamily, homolog |
| ID2 | NM_002166 | Inhibitor of DNA binding 2 |
| LAMA2 | NM_000426 | Laminin, alpha 2 |
| MGP | NM_000900 | Matrix Gla protein |
| MMP1 | NM_002421 | Matrix metallopeptidase 1 |
| MMP3 | NM_002422 | Matrix metallopeptidase 3 |
| SFRP2 | NM_003013 | Secreted frizzled-related protein 2 |
| TNC | NM_002160 | Tenascin C |
| WISP2 | NM_003881 | WNT1 inducible signaling pathway protein 2 |
| WNT5A | NM_003392 | Wingless-type MMTV integration site family, member 5A |

**TABLE 6 : Applied Biosystems Taqman Gene Expression Assay**

| gene name | TaqMan® Gene Expression Assay Reference |
|---|---|
| MMP1 | Hs00233958_m1 |
| MMP3 | Hs00233962_m1 |
| COL3A1 | Hs00943809_m1 |
| COL7A1 | Hs00164310_m1 |
| COL11A1 | Hs00266273_m1 |
| LAMA2 | Hs00166308_m1 |
| FGF7 | Hs00940253_m1 |
| GREM1 | Hs00171951_m1 |
| MGP | Hs00179899_m1 |
| CXCL12 | Hs00171022_m1 |
| ANGPTL2 | Hs00765775_m1 |
| ANGPTL4 | Hs00211522_m1 |
| WNT5A | Hs00180103_m1 |
| SFRP2 | Hs00293258_m1 |
| DKK3 | Hs00247426_m1 |
| ID2 | Hs00747379_m1 |
| WISP2 | Hs00180242_m1 |
| TNC | Hs01115664_m1 |
| TBP | Hs99999910_m1 |
| GAPDH | Hs99999905_m1 |
| PPIA | Hs99999904_m1 |
| RPLO1 | Hs99999902_m1 |
| B2M | Hs99999907_m1 |

## Claims

1. An *in vitro* method for detecting a predisposition to basal cell carcinoma in a human subject, said method comprising i) providing a biological sample of said human subject; ii) determining in said sample the amount of gene products for at least 11 genes from the group consisting of MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC; iii) comparing the determined amounts to reference amounts, thereby detecting the human subject which has a predisposition to a basal cell carcinoma.

2. Method of claim 1, wherein MMP1, MMP3, COL11A1, CXCL12, MGP, ANGPTL2, ANGPTL4, FGF7, GREM1, SFRP2, WISP2, ID2 and TNC are over-expressed and COL3A1, COL7A1, LAMA2, DKK3 and WNT5A are under-expressed in a human subject having a predisposition to nevoid basal cell carcinoma syndrome (NBCCS) or to a basal cell carcinoma in comparison to a healthy control.

3. Method of claim 1 or 2, the biological sample comprises fibroblasts, preferably a skin biopsy.

4. Method according to anyone of claims 1-3, wherein the predisposition to a familial basal cell carcinoma or to a nevoid basal cell carcinoma syndrome is detected.

5. Method according to anyone of claims 1-4, wherein the method comprises the determination of the amount of gene products for at least the following genes MMP1, MMP3, COL11A1, LAMA2, CXCL12, MGP, ANGPTL2, ANGPTL4, FGF7, SFRP2 and DKK3.

6. Method according to anyone of claims 1-5, wherein the method comprises the determination of the amount of gene products for at least the following genes : MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.

7. Method according to anyone of claims 1-6, wherein the method further comprises the determination of the amount of a gene product selected in the group consisting of the Table 1, preferably of Table 3.

8. An *in vitro* method for screening or identifying a compound useful for treating a basal cell carcinoma, comprising i) providing fibroblasts having an expression profile with the genes MMP1, MMP3, COL11A1, CXCL12, MGP, ANGPTL2, ANGPTL4, FGF7, GREM1, SFRP2, WISP2, ID2 and TNC over-expressed and the genes COL3A1, COL7A1, LAMA2, DKK3, and WNT5A under-expressed; ii) contacting a test compound with the fibroblast; iii) determining the expression level of the genes MMP1, MMP3, COL11A1, CXCL12, MGP, ANGPTL2, ANGPTL4, FGF7, GREM1, SFRP2, WISP2, ID2, TNC, COL3A1, COL7A1, LAMA2, DKK3, and WNT5A; and iv) selecting the test compound which decreases the expression of at one gene selected in the group consisting of MMP1, MMP3, COL11A1, CXCL12, MGP, ANGPTL2, ANGPTL4, FGF7, GREM1, SFRP2, WISP2, ID2 and TNC and/or which increases the expression of at one gene selected in the group consisting of COL3A1, COL7A1, LAMA2, DKK3, and WNT5A in the fibroblasts.

9. Method according to claim 8, wherein the fibroblasts have a non-sense or missense mutation of the gene *PACTHED*.

10. Method according to anyone of claims 1-9, wherein the gene product is mRNA and preferably the amount of mRNA is determined by real-time quantitative or semi-quantitative RT-PCR or with a DNA chip.

11. Method according to anyone of claims 1-9, wherein the gene product is a polypeptide and preferably the amount of polypeptide is determined by quantitative immunoassay, such as ELISA.

12. A kit comprising a set of detection means selected from the group consisting of a pair of primers, a probe and an antibody specific to at least 11 of the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.

13. A DNA chip comprising a solid support which carries nucleic acids that are specific to at least 11 of the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.

14. Kit of claim 12 or DNA chip of claim 13, wherein thedetection means or nucleic acids are specific to at least the following genes MMP1, MMP3, COL11A1, LAMA2, CXCL12, MGP, ANGPTL2, ANGPTL4, FGF7, SFRP2 and DKK3.

15. Kit of claim 12 or DNA chip of claim 13, wherein the detection means or nucleic acids are specific to at least the following genes MMP1, MMP3, COL3A1, COL7A1, COL11A1, LAMA2, CXCL12, MGP, ANGPTL2, ANGPTL4, FGF7, GREM1, SFRP2, DKK3, WNT5A, WISP2, ID2 and TNC.
